# FASCICULE DE BREVET EUROPEEN

(11) **EP 2 280 936 B1**
(45) Date de publication et mention de la délivrance du brevet: **15.08.2018**
(21) Numéro de dépôt: 09738225.3
(22) Date de dépôt: 30.04.2009
(51) Int. Cl.: C07C 235/06

(54) **COMPOSES DE TYPE ETHER-AMIDE, PROCEDE DE PREPARATION ET UTILISATIONS**
VERBINDUNGEN VOM ETHERAMIDTYP, HERSTELLUNGSVERFAHREN UND ANWENDUNGEN
ETHER-AMIDE TYPE COMPOUNDS, PREPARATION METHOD AND USES

(30) Priorité: 30.04.2008 FR 0802433
(43) Date de publication de la demande: 09.02.2011
(73) Titulaire: Rhodia Opérations, 93306 Aubervilliers (FR)
(72) Inventeur: GUGLIERI, Massimo, MC- 98000 Monaco (MC); JENTZER, Olivier, Vourles 69390 (FR)
(74) Mandataire: Cardon, Flavie
(86) Numéro de dépôt international: PCT/EP2009/055287
(87) Numéro de publication internationale: WO 2009/133181

(56) Documents cités:
- FR-A1- 2 312 253
- FR-A1- 2 385 686
- JP-A- 2005 047 885
- JP-A- 2008 031 112
- US-A- 2 704 280

## Description

La présente invention a pour objet de nouveaux composés, de type éther amide. Elle a également pour objet un procédé pour la préparation de tels composés. Elle a également pour objet des utilisations de tels composés, notamment comme solvants, par exemple dans des formulations phytosanitaires.

L'industrie utilise de nombreux composés chimiques à titre de solvants, par exemple pour préparer des produits chimiques et des matériaux, pour formuler des composés chimiques, ou pour traiter des surfaces. Par exemple des solvants sont utilisés pour la formulation d'actifs phytosanitaires notamment sous forme de concentrés émulsionnables (Emulsifiable Concentrate "EC") destinés à être dilués dans de l'eau par l'exploitant agricole, avant application sur un champ.

L'industrie est à la recherche de nouveaux composés permettant de varier ou d'optimiser les produits et procédés dans lesquels des solvants, notamment des solvants polaires, sont à utiliser. L'industrie a notamment besoin de composés de coût modeste, présentant des propriétés d'usage intéressantes. L'industrie a également besoin de composés présentant un profile toxicologique et/ou écologique perçu comme favorable, notamment une faible volatilité (faible VOC), une bonne biodégradabilité, une faible toxicité et/ou une faible dangerosité.

On connaît l'utilisation comme solvants des dialkylamides. Il s'agit de produit de formule R-CONMe₂ ou R est un groupe hydrocarboné comme un alkyle, typiquement en C₆-C₃₀. De tels produits sont notamment commercialisés sous la dénomination Genagen® par la société Clariant. Ces solvants trouvent des applications notamment dans le domaine phytosanitaire. Ces solvants présentent toutefois un domaine d'utilisation restreint et ne permettent pas de solubiliser certains actifs phytosanitaires à certaines concentrations, dans des gammes de températures utiles, sans formation de cristaux.

JP 2005 047 885 décrit des composés de formule R¹-O-CH₂-CH₂-CONR²R³, pouvant être utilisés comme solvants et JP 2008 031 112 décrit des composés de formule R¹-O-CH₂-CH₂-CO-NH-CHMe₂, utilisés comme solvants. Il demeure un besoin pour de nouveaux solvants, notamment dans les formulations phytosanitaires, et pour de nouveaux composés, pouvant notamment élargir la gamme d'actifs pouvant être formulés et/ou leur concentration et/ou pouvant élargir les conditions d'utilisation, notamment en terme de stabilité par exemple sans formation de cristaux, à basse température.

La présente invention répond à au moins un des besoins exprimés ci-dessus en proposant un composé de formule (I) suivante :

R^{a}R^{b}C(OR¹)-CHR^{c}-CONR²R³ (I)

où
- R^{a}, R^{b} et R^{c}, identiques ou différents, sont des groupes choisis parmi l'atome d'hydrogène et les groupes alkyles linéaires ou ramifiés, de préférence en C₁-C₃, au moins un des groupes choisis parmi R^{a}, R^{b} et R^{c} étant différent de l'atome d'hydrogène,
- R¹ est un groupe choisi parmi les groupes méthyle, éthyle, propyle, isopropyle, n-butyle, isobutyle, n-pentyle, isopentyle, isoamyle, n-hexyle, cyclohexyle, n-octyle, isooctyle, 2-ethylhexyle ou tridécyle ;
- R² et R³, identiques ou différents, sont des groupes choisis les groupes méthyle, éthyle, n-propyle, isopropyle, n-butyle, isobutyle, n-pentyle, amyle, isoamyle, hexyle ou cyclohexyle.

L'invention a également pour objet un procédé de préparation du composé. L'invention a également pour objet l'utilisation du composé comme tensioactif, solvant, co-solvant, décapant, inhibiteur de cristallisation, agent nettoyant, agent de dégraissage, agent plastifiant ou agent de coalescence. L'invention a également pour objet un procédé de solvatation, co-solvatation, plastification, coalescence et/ou inhibition de cristallisation par ajout du composé de l'invention. L'invention a également pour objet des formulations comprenant le composé de l'invention. Les formulations peuvent être notamment des formulations phytosanitaires.

### Définitions ou abréviations

Dans la présente demande on utilise notamment les abréviations suivantes: Me signifie méthyle; Et signifie éthyle; IsoAm signifie Isoamyle, cyclo signifie cyclohexyle.

Dans la présente demande, par "composition de matière", on entend une composition, plus ou moins complexe, comprenant plusieurs composés chimiques. Il peut s'agir typiquement d'un produit de réaction non purifié ou modestement purifié. Le composé de l'invention pourra notamment être isolé et/ou commercialisé et/ou utilisé sous forme d'une composition de matière le comprenant. Le composé de l'invention, sous forme de molécule pure ou sous forme d'un mélange répondant à la formule (I), peut être compris dans une composition de matière.

Dans la présente demande le terme solvant est entendu dans un sens large, couvrant notamment les fonctions de co-solvant, d'inhibiteur de cristallisation, de décapant. Le terme solvant peut notamment désigner un produit liquide à la température d'utilisation, de préférence de point de fusion inférieur ou égal à 20°C, de préférence à 5°C, de préférence à 0°C, pouvant contribuer à rendre liquide une matière solide, ou à empêcher ou retarder la solidification ou la cristallisation de matière dans un milieu liquide.

### Composé de l'invention

Le composé de l'invention présente la formule générale (I) donnée ci-dessus. On note qu'il peut s'agir d'un mélange de plusieurs composés de formule générale (I). En d'autres termes le composé peut être seul ou en mélange. Dans le cadre de mélanges de plusieurs composés les nombres d'atomes ou de motifs peuvent être exprimés en nombre moyens. Il s'agit de nombres moyens en nombre. Dans le cas de composés seuls, il s'agira généralement de nombres entiers, en ce qui concerne le nombre d'atomes de carbone.

Les groupes R^{a}, R^{b} et R°, identiques ou différents, sont des groupes choisis parmi l'atome d'hydrogène et les groupes alkyles linéaires ou ramifiés. Les alkyles peuvent notamment être des alkyles en C₁-C₆, de préférence en C₁-C₃. Il peut notamment s'agir de groupes méthyle ou éthyle. Selon un mode de réalisation particulier, nombre total d'atomes de carbones, hors groupes R¹, R² et R³ est de 4, 5, ou 6. Au moins un des groupes choisis parmi R^{a}, R^{b} et R^{c} est différent de l'atome d'hydrogène, par exemple un groupe choisi parmi les groupes alkyles linéaires ou ramifiés. Les alkyles peuvent notamment être des alkyles en C₁-C₆, de préférence en C₁-C₃. Il peut notamment s'agir de groupes méthyle ou éthyle. Selon un mode de réalisation particulier, nombre total d'atomes de carbones, hors groupes R¹, R² et R³ est de 4, 5, ou 6.

Selon un mode de réalisation particulier R^{c} est un groupe méthyle, et R^{a} et R^{b}, identiques ou différents, peuvent être des groupes choisis parmi l'atome d'hydrogène et les groupes alkyles linéaires ou ramifiés. Les alkyles peuvent notamment être des alkyles en C₁-C₆, de préférence en C₁-C₃. Il peut notamment s'agir de groupes méthyle ou éthyle. Selon un mode de réalisation particulier, nombre total d'atomes de carbones, hors groupes R¹, R² et R³ est de 4, 5, ou 6.

Selon un mode de réalisation particulier R^{c} est un atome d'hydrogène, et R^{a} est un groupe choisi parmi l'atome d'hydrogène et les groupes alkyles linéaires ou ramifiés, et R^{b} est un groupe choisi parmi les groupes alkyles linéaires ou ramifiés. Les alkyles peuvent notamment être des alkyles en C₁-C₆, de préférence en C₁-C₃. Il peut notamment s'agir de groupes méthyle ou éthyle. Selon un mode de réalisation particulier, nombre total d'atomes de carbones, hors groupes R¹, R² et R³ est de 4, 5, ou 6.

On mentionne que R^{a} peut être un groupe choisis parmi les groupes alkyles linéaires ou ramifiés. Les alkyles peuvent notamment être des alkyles en C₁-C₆, de préférence en C₁-C₃. Il peut notamment s'agir de groupes méthyle ou éthyle. Selon un mode de réalisation particulier, nombre total d'atomes de carbones, hors groupes R¹, R² et R³ est de 4, 5, ou 6.

On mentionne que R^{b} et R^{c}, identiques ou différents, peuvent être des groupes choisis parmi l'atome d'hydrogène et les groupes alkyles linéaires ou ramifiés. Les alkyles peuvent notamment être des alkyles en C₁-C₆, de préférence en C₁-C₃. Il peut notamment s'agir de groupes méthyle ou éthyle. Selon un mode de réalisation particulier, nombre total d'atomes de carbones, hors groupes R¹, R² et R³ est de 4, 5, ou 6.

Selon un mode de réalisation particulier:
- R^{a} est un groupe choisis parmi les groupes alkyles linéaires ou ramifiés. Les alkyles peuvent notamment être des alkyles en C₁-C₆, de préférence en C₁-C₃. Il peut notamment s'agir de groupes méthyle ou éthyle. Selon un mode de réalisation particulier, nombre total d'atomes de carbones, hors groupes R¹, R² et R³ est de 4, 5, ou 6.
- R^{b} et R^{c}, identiques ou différents, sont des groupes choisis parmi l'atome d'hydrogène et les groupes alkyles linéaires ou ramifiés. Les alkyles peuvent notamment être des alkyles en C₁-C₆, de préférence en C₁-C₃. Il peut notamment s'agir de groupes méthyle ou éthyle. Selon un mode de réalisation particulier, nombre total d'atomes de carbones, hors groupes R¹, R² et R³ est de 4, 5, ou 6.

Selon des modes de réalisation particulier, R^{c}= H et R^{b} = H ou R^{c}= Me et R^{b} = H, ou R^{c} = H et R^{b} = Me. Selon des modes de réalisation particuliers R^{a} est un groupe méthyle ou éthyle.

Selon un mode de réalisation particulier, R^{c}= H, et R^{b} = H, et R^{a} = Et. Ce mode de réalisation particulier peut être par exemple mis en oeuvre par transformation à partir d'un un alcène-nitrile de formule (I') de type 2-pentènenitrile (parfois noté 2PN). Il peut s'agir notamment de cis-2-pentènenitrile ou de trans-2-pentènenitrile.

Selon un autre mode de réalisation particulier, R^{c} = Me et R^{b} = H et R^{a} = Me ou R^{c} = H et R^{b} = Me et R^{a} = Me. Ce mode de réalisation particulier peut être par exemple mis en oeuvre par transformation à partir d'un alcène-nitrile de formule (I') de type méthyle-2-butènenitrile (parfois noté 2BN). Il peut s'agir notamment d'un 2-méthyle-2-butènenitrile comme le cis-2-méthyle-2-butènenitrile ou le trans-2-méthyle-2-butènenitrile, ou d'un 3-méthyle-2-butènenitrile comme le cis-3-méthyle-2-butènenitrile ou le trans-3-méthyle-2-butènenitrile.

Selon un mode de réalisation particulier le composé est un mélange de composés tels que R^{c}= H, et R^{b} = H, et R^{a} = Et pour le premier, et R^{c} = Me et R^{b} = H et R^{a} = Me pour le deuxième. Le rapport molaire entre le premier et le deuxième peut par exemple être compris entre 50/50 et 99/1, de préférence entre 60/40 et 90/10.

R² et R³ peuvent notamment être des groupes méthyle, de préférence tous les deux.

Le groupe R¹ est un groupe typiquement correspondant à un alcool R¹-OH.

Selon un mode de réalisation particulier R¹ est un groupe différent du n-hexyle et R^{a} et/ou R^{b} est différent de l'atome d'hydrogène. Selon un mode de réalisation particulier R¹ est un groupe différent du n-butyle et R^{a} et/ou R^{b} est différent de l'atome d'hydrogène. Selon un mode de réalisation particulier R¹ est un groupe différent de l'éthylhexyle et R^{a} et/ou R^{b} est différent de l'atome d'hydrogène.

Selon un mode de réalisation particulier R¹ est un groupe différent du n-hexyle. Selon un mode de réalisation particulier R¹ est un groupe différent du n-butyle. Selon un mode de réalisation particulier R¹ est un groupe différent de l'éthylhexyle.

Selon un mode de réalisation particulier le groupe R¹ est un groupe cyclohexyle. Les composés présentant un tel groupe présentent des propriétés solvantes et/ou des propriétés de miscibilité dans l'eau particulièrement intéressantes, notamment dans le cadre de formulations phytosanitaires.

Le composé de l'invention est de préférence tel qu'il présente un point de fusion inférieur à ou égal à 20°C, de préférence à 5°C, de préférence à 0°C. Les groupes détaillés ci-dessus sont de préférence tels que le composé présente cette propriété.

Selon un mode de réalisation le composé de l'invention peut être totalement miscible dans l'eau. Selon un mode de réalisation particulier, le composé de l'invention est partiellement miscible dans l'eau. La miscibilité dans l'eau peut par exemple être inférieure à 10% en poids (à 25°C), de préférence à 2%, de préférence à 1% ou à 0,1%. Elle peut être supérieure à 0,001%, de préférence à 0,01% ou à 0,1%. Elle peut par exemple être comprise entre 0,01% et 2%, par exemple entre 0,1% et 1%. De manière surprenante les composés de l'invention présentent de bonnes propriétés solvantes, notamment pour des actifs phytosanitaires dans des formulations phytosanitaires avec une faible miscibilité dans l'eau. Les groupes R^{a}, R^{b}, R^{c}, et/ou le groupe R¹ et/ou les groupes R², R³ peuvent être choisis de manière à contrôler la miscibilité dans l'eau.

Le composé de l'invention présente de préférence l'une des formules suivantes:

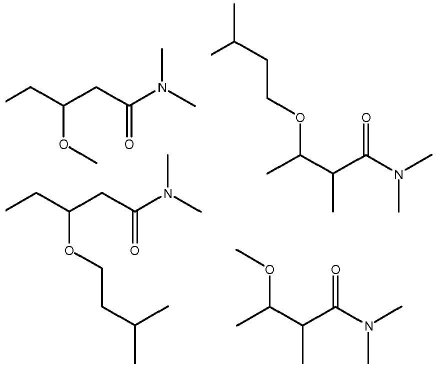

On mentionne que le composé peut être un mélange de composés présentant ces formules. Il peut s'agir par exemple des mélanges suivants: 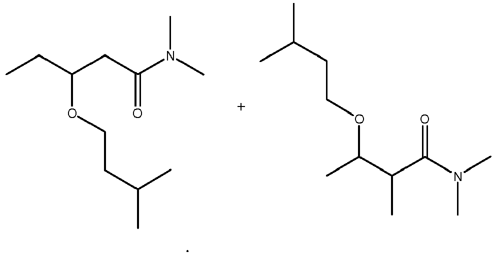 ou

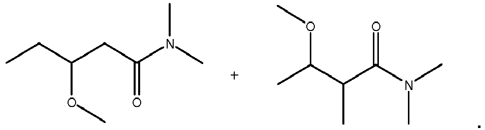

On mentionne que le composé de l'invention peut être compris dans une composition de matière, comprenant d'autres produits que le composé seul ou en mélange répondant à la formule (I). Dans la composition de matière le composé de l'invention peut représenter au moins 10% en poids. De préférence, il s'agit du composé principal de la composition de matière. Par composé principal, on entend dans la présente demande, le composé dont la teneur est la plus élevée, même si sa teneur est inférieure à 50% en poids (par exemple dans un mélange de 40% de A, de 30% de B, et de 30% de C, le produit A est le composé principal). Encore plus préférablement le composé de l'invention représente au moins 50% en poids de la composition de matière, par exemple de 70 à 95% en poids, et même de 75 à 90% en poids. Comme indiqué plus haut, la composition de matière peut être un produit de réaction. Les autres produits de la composition de matière peuvent notamment être des sous-produits des impuretés, des produits n'ayant pas réagi, ou des produits correspondant à des adduits de réaction de produits compris dans les composés de départ ne menant pas aux composés de formule (I).

### Procédé

Le composé de l'invention peut notamment être préparé par transformation à partir d'un alcène-nitrile de formule (I')

R^{a}R^{b}C=CR^{c}-CN (I').

On note que dans tous les procédés et séquences mentionnés ci-dessous, on peut mettre en oeuvre des étapes optionnelles intermédiaires de séparation et/ou de purification, afin par exemple d'éliminer des sous-produits non visés. Les sous-produits peuvent être éventuellement utilisés pour fabriquer d'autres produits, ou peuvent être transformés afin d'être réintroduits dans le procédé. Le procédé peut être suivi d'étapes de filtration et/ou de purification par exemple par distillation.

On peut notamment opérer une transformation par un procédé comprenant une transformation par addition d'un alcool de formule R¹-OH sur la double liaison, et une transformation du groupe -CN en groupe -CONR²R³. Ces transformations peuvent être opérées dans n'importe quel ordre. On préfère toutefois opérer l'addition de l'alcool avant la transformation du groupe -CN.

L'addition de l'alcool peut être réalisée de manière connue, par exemple en présence d'une base, souvent en quantité catalytique. Il s'agit d'une addition de type Michael. Elle peut être mise en oeuvre dans un solvant, de préférence dans un solvant non aqueux. A titre de solvant on peut avantageusement utiliser l'alcool de formule R¹-OH, alors en excès.

La transformation du groupe -CN peut notamment comprendre une transformation du groupe -CN en groupe -COOH ou -COOR' où R' est un alkyle en C₁-C₄, puis une transformation du groupe -COOH ou -COOR' en groupe -CONR²R³ à l'aide d'une amine de formule HNR²R³.

Dans un mode de réalisation le procédé comprend les étapes suivantes:
étape 1) on réagit l'alcène-nitrile de formule (I') avec un alcool de formule R¹-OH pour obtenir un éther-nitrile de formule (II')

   R^{a}R^{b}C(OR¹)-CHR^{C}-CN (II')
étape 2) on transforme le groupe -CN de l'éther-nitrile en groupe amide de manière à obtenir le produit de formule (I).

L'étape 1) constitue typiquement une réaction de Michael. Comme mentionné ci-dessus, elle peut être mise en oeuvre en présence d'une base, de préférence dans un solvant non aqueux, par exemple l'alcool de formule R¹-OH, typiquement en excès large. A titre de base on peut par exemple mettre en oeuvre un hydroxyde d'ammonium, un métal alcalin etc...

L'étape 2) constitue typiquement une transformation du groupe -CN en groupe - CONR²R³. L'étape 2) comprend avantageusement les étapes suivantes:
étape 2a): on transforme le groupe -CN en groupe -COOH ou -COOR' où R' est un alkyle en C₁-C₄,
étape 2b): on transforme le groupe -COOH ou -COOR' en groupe -CONR²R³ à l'aide d'une amine de formule HNR²R³, de manière à obtenir le composé de formule (I).

Selon un mode particulier de réalisation l'étape 2) comprend les étapes suivantes:
2a') on transforme le groupe -CN en groupe -COOH directement par hydrolyse ou en formant un groupe -COOR' puis en hydrolysant,
2b') on transforme le groupe -COOH en groupe -CONR²R³ directement par réaction avec une amine de formule HNR²R³ ou en formant un groupe -COCl puis en réagissant avec une amine de formule HNR²R³, de manière à obtenir le composé de formule (I).

On peut notamment mettre en oeuvre la séquence suivante:
2a'1) on transforme le groupe -CN en groupe -COOH par hydrolyse
2b'1) on transforme le groupe -COOH en groupe -CONR²R³ directement par réaction avec une amine de formule HNR²R³.

On peut alternativement mettre en oeuvre la séquence suivante:
2a'1) on transforme le groupe -CN en groupe -COOH par hydrolyse
2b'2) on forme un groupe -COCl
2b'3) on réagit avec une amine de formule HNR²R³.

On peut alternativement mettre en oeuvre la séquence suivante:
2a'2) on forme un groupe -COOR'
2a'3) on hydrolyse en groupe -COOH
2b'1) on transforme le groupe -COOH en groupe -CONR²R³ directement par réaction avec une amine de formule HNR²R³.

On peut alternativement mettre en oeuvre la séquence suivante:
2a'2) on forme un groupe -COOR'
2a'3) on hydrolyse en groupe -COOH
2b'2) on forme un groupe -COCl
2b'3) on réagit avec une amine de formule HNR²R³.

Selon un autre mode particulier de réalisation l'étape 2) comprend les étapes suivantes:
2a") on transforme le groupe -CN en groupe -COOR'
2b") on transforme le groupe -COOR' en groupe -CONR²R³ à l'aide d'une amine de formule HNR²R³, de manière à obtenir le composé de formule (I).

L'étape 2b") est une réaction de trans-amidification. Elle peut être mise en oeuvre de manière connue. Lors de cette réaction on met de préférence en oeuvre de 0,8 à 1,2 moles, de préférence de 0,9 à 1,1 moles, de préférence environ 1 mole, d'amine par mole d'ester. Elle peut notamment être mise en oeuvre à l'aide de catalyseurs acides ou basiques, par exemple à l'aide carbonate de potassium ou d'ortho titanates d'alkyles. Cette étape peut être mise en oeuvre en solution, par exemple en solution aqueuse, ou en solution dans du toluène. On peut au cours de cette étape éliminer progressivement le méthanol formé afin de favoriser la réaction. L'élimination peut s'accompagner d'une élimination du solvant, par exemple à un azéotrope. Après séparation du méthanol le solvant éliminé peut être réintroduit dans le procédé.

Les étapes 2a") et 2a'2) sont des réactions d'estérification à partir d'un nitrile. Elles peuvent être mises en oeuvre de manière connue. On peut notamment mettre en oeuvre la réaction de PINNER. Elles peuvent être mises en oeuvre à l'aide d'un alcool de formule R'OH, de préférence en excès. Cet alcool peut constituer le solvant de la réaction.

L'étape 2a'1) est une réaction d'hydrolyse. Elle peut être mise en oeuvre de manière connue, notamment par hydrolyse acide ou hydrolyse basique.

L'étape 2b'2) est une réaction connue. Elle peut notamment être mise en oeuvre à l'aide de chlorure de thionyle. Elle s'accompagne de formation d'acide chlorhydrique. On peut utiliser une base afin de le piéger, par exemple de la triéthylamine (TEA). Cette étape peut être mise en oeuvre avec au moins 0.8 équivalent molaire d'amine, de préférence avec au moins un équivalent. On peut notamment mettre en oeuvre un excès de 1,05 à 1,4 équivalents molaires.

L'addition de l'alcool, notamment l'étape 1), est de préférence mise en oeuvre à l'aide d'au moins 1 équivalent molaire d'alcool, par rapport à l'alcène-nitrile. On peut mettre en oeuvre un fort excès d'alcool, par exemple de 2 à 20 équivalents, notamment de 5 à 15. On peut notamment utiliser l'alcool comme solvant de la réaction.

A titre de composés alcène nitrile de départ, on peut notamment mettre en oeuvre les alcène-nitrile pour lesquels:
- R^{c}= H, et R^{b} = H, et R^{a} = Et. Il s'agit d'un alcène-nitrile de formule (I') de type 2-pentènenitrile (parfois noté 2PN). Il peut s'agir notamment de cis-2-pentènenitrile ou de trans-2-pentènen itrile.
- R^{c} = Me et R^{b} = H et R^{a} = Me ou R^{c} = H et R^{b} = Me et R^{a} = Me. Il peut s'agir d'un alcène-nitrile de formule (I') de type méthyle-2-butènenitrile (parfois noté 2BN). Il peut s'agir notamment d'un 2-méthyle-2-butènenitrile comme le cis-2-méthyle-2-butènenitrile ou le trans-2-méthyle-2-butènenitrile, ou d'un 3-méthyle-2-butènenitrile comme le cis-3-méthyle-2-butènenitrile ou le trans-3-méthyle-2-butènenitrile.

On peut notamment mettre en oeuvre un mélange d'alcène-nitrile où R^{c}= H, et R^{b} = H, et R^{a} = Et pour le premier, et R^{c} = Me et R^{b} = H et R^{a} = Me pour le deuxième. Le rapport molaire entre le premier et le deuxième peut par exemple être compris entre 50/50 et 99/1, de préférence entre 60/40 et 90/10. On peur notamment mettre en oeuvre un mélange de cis-2-pentènenitrile (le premier) et de trans-2-méthyle-2-butènenitrile (le deuxième), par exemple dans rapport molaire entre le premier et le deuxième compris entre 50/50 et 99/1, de préférence entre 60/40 et 90/10.

Le composé de l'invention peut alternativement être préparé par transformation à partir d'un composé insaturé de formule suivante R^{a}R^{b}C=CR^{c}-CO-NR¹R², par example à partir de composés acrylamidiques (où R^{C}=H) ou de methacrylamidiques (où R^{C}=CH₃). On peut par exemple opérer par addition de Michaël, par réaction d'un alcool ou d'un alcoolate de formule R¹-OH ou R¹-O⁻ sur la double liaison. De telles réactions sont connues de l'homme du métier. Elles peuvent être mises en oeuvre en présence d'un catalyseur basique.

Le composé de l'invention peut alternativement être préparé par transformation à partir d'un composé insaturé de formule suivante R^{a}R^{b}C=CR^{c}-COOH ou R^{a}R^{b}C=CR^{c}-COOR' ou R^{a}R^{b}C=CR^{c}-CONH₂. On peut par exemple opérer une addition de Michaël puis transformer le groupe -COOH ou -COOR' ou -CONH₂ en groupe - CONR²R³ par amidification ou transamidification ou réaction de substitution. On peut alternativement transformer le groupe -COOH ou -COOR' ou -CONH₂ en groupe - CONR²R³ par amidification ou transamidification ou réaction de substitution puis opérer une addition de Michaël.

### Utilisations - Formulations

Le composé de l'invention peut notamment être utilisé comme tensioactif, solvant, co-solvant et/ou inhibiteur de cristallisation, comme agent plastifiant ou comme agent de coalescence.

Par co-solvant, on entend que d'autres solvants peuvent lui être associés. L'utilisation à titre de solvant ou de co-solvant comprend notamment des utilisations pour dissoudre un composé dans une formulation, dans un milieu réactionnel, l'utilisation pour solubiliser totalement ou partiellement un produit à éliminer (dégraissage, décapage), et/ou pour faciliter de le décollage de films de matières.

Pour l'utilisation à titre de tensioactif, on préfère les composés alcoxylés et/ou propoxylés, c'est-à-dire où n est différent de 0 dans la formule (I).

Le composé de l'invention peut notamment être utilisé, pour les fonctions indiquées ci-dessus ou pour d'autres, dans une formulation phytosanitaire, dans une formulation de nettoyage, dans une formulation de décapage, dans une formulation de dégraissage, dans une formulation de lubrifiants ou textiles, dans une formulation de revêtement, par exemple dans une formulation de peinture, dans une formulation de pigments ou encre, dans une formulation plastique.

Le composé peut par exemple être utilisé à titre d'agent de coalescence dans une formulation de peinture aqueuse.

Le composé peut notamment être utilisé comme solvant de résines par exemple dans l'industrie du revêtement de câbles ou dans l'industrie électronique, notamment comme solvant du PVDF.

Le composé peut notamment être utilisé comme solvant de nettoyage et/ou de décapage dans l'industrie électronique. Il peut notamment être utilisé dans des batteries au Lithium. Il peut notamment être utilisé sur des résines photorésistantes, des polymères, des cires des graisses, des huiles.

Le composé peut notamment être utilisé pour le nettoyage d'encres, par exemple lors de la production d'encres ou lors de l'utilisation d'encre en impression.

Le composé peut notamment être utilisé pour ne nettoyage de tamis ou d'autres outils mis en oeuvre dans des procédés de fabrication et/ou de recyclage de papier.

Le composé peut notamment être utilisé pour le nettoyage de bitumes ou de sables bitumineux ("tar sand" en anglais), par exemple sur les substrats revêtus, sur les outils utilisés pour appliquer ces matières, sur des vêtements souillés, sur des véhicules souillés.

Le composé peut notamment être utilisé pour le nettoyage d'engins volants comme des avions, des hélicoptères, des navettes spatiales.

Le composé peut notamment être utilisé comme agent de dégraissage sur des surfaces métalliques, par exemple des surfaces d'outils, d'objets manufacturés, de toles, de moules, notamment en acier ou en aluminium ou en alliages de ces métaux.

Le composé peut notamment être utilisé comme solvant de nettoyage sur des surfaces dures ou des surfaces textiles.

Le composé peut notamment être utilisé comme solvant de décapage de peinture ou de résines, sur des surfaces d'outils, par exemple des moules de fonderie, sur des surfaces des sites industriels (sols, cloisons etc...). Les formulations de décapage de peintures peuvent notamment être des formulations à base aqueuse (le composé étant en mélange avec de l'eau) ou à base solvant (le composé étant alors le solvant ou un composé en mélange avec de l'eau).

Le composé peut notamment être utilisé comme agent plastifiant dans des formulations de polymères thermoplastiques.

Les formulations de nettoyage et/ou de dégraissage peuvent notamment être des formulations pour les soins ménagers, opérés dans les foyers ou dans les domaines publiques (hotels, bureaux, usines....). Il peut s'agir de formulation pour le nettoyage des surfaces dures comme les sols, les surfaces d'ameublement et d'équipement des cuisines et salles de bain, la vaisselle. Ces formulations peuvent également être utilisées dans la sphère industrielle pour dégraisser des produits manufacturés et/ou les nettoyés. De telles formulation peuvent notamment être utilisées pour nettoyer et/ou décaper des produits, des outils, des moules, des habits ou autres.

Le composé de l'invention peut notamment être utilisé dans des formulations phytosanitaires comprenant un produit actif solide. Plus de détails sont donnés ci-dessous, où le mot "solvant" peut désigner le composé de l'invention ou une composition de matière le comprenant, décrite ci-dessus.

### Utilisation détaillée dans le cadre de formulations phytosanitaires

La formulation phytosanitaire est généralement une formulation phytosanitaire concentrée comprenant un composé actif.

L'agriculture utilise de nombreuses matières actives telles que des fertilisants ou des pesticides, par exemple des insecticides, herbicides ou fongicides. On parle de produits phytosanitaires actifs (ou de matière active). Les produits phytosanitaires actifs sont généralement produits sous forme pure ou très concentrée. Ils doivent être utilisés sur les exploitations agricoles à de faibles concentrations. A cette fin, ils sont généralement formulés avec d'autres ingrédients afin de permettre une dilution en poids aisée par l'exploitant agricole. On parle de formulations phytosanitaires. La dilution opérée par l'exploitant agricole est généralement réalisée par mélange de la formulation phytosanitaire avec de l'eau.

Ainsi les formulations phytosanitaires doivent permettre une dilution en poids aisée par l'exploitant agricole, afin d'obtenir un produit dans lequel le produit phytosanitaire est correctement dispersé, par exemple sous forme de solution, d'émulsion, de suspension, ou de suspo-émulsion. Les formulations phytosanitaires permettent ainsi le transport d'un produit phytosanitaire sous forme relativement concentrée, un conditionnement aisé et/ou une manipulation aisée pour l'utilisateur final. Différents types de formulations phytosanitaires peuvent être utilisés selon les différents produits phytosanitaires. On cite par exemple les concentrés émulsionnables (Emulsifiable Concentrates «EC»), les émulsions concentrées (Emulsion in water "EW"), les microémulsions ("ME"), les poudres mouillables (Wettable Powders «WP»), les granulés dispersables dans l'eau (Water Dispersible Granules, «WDG»). Les formulations qu'il est possible d'utiliser dépendent de la forme physique du produit phytosanitaire (par exemple solide ou liquide), et de ses propriétés physico-chimiques en présence d'autres composés comme l'eau ou les solvants.

Après dilution en poids par l'exploitant agricole, par exemple par mélange avec de l'eau, le produit phytosanitaire peut se trouver sous différentes formes physiques: solution, dispersion de particules solides, dispersion de gouttelettes du produit, gouttelettes de solvant dans lequel le produit est dissous... Les formulations phytosanitaires comprennent généralement des composés permettant d'obtenir ces formes physiques. Il peut par exemple s'agir de tensioactifs, de solvants, de supports minéraux, et/ou de dispersants. Bien souvent ces composés n'ont pas un caractère actif, mais un caractère d'intermédiaire d'aide à la formulation. Les formulations phytosanitaires peuvent notamment être sous forme liquide, ou sous forme solide.

Afin de préparer des formulations phytosanitaires de produits phytosanitaires actifs solides, il est connu de solubiliser le produit dans un solvant. La formulation phytosanitaire comprend ainsi une solution du produit dans le solvant. La formulation peut être sous forme solide, par exemple sous forme de poudre mouillable (WP) où la solution imbibe un support inorganique, par exemple du kaolin et/ou de la silice. La formulation peut alternativement être sous forme liquide, par exemple sous forme de concentré émulsionnable (EC) présentant une seule phase liquide limpide comprenant le solvant et le produit en solution, pouvant former une émulsion par ajout d'eau, sans agitation ou avec une faible agitation. Elle peut aussi être ou sous forme d'une émulsion concentrée (EW), trouble, dont la phase dispersée dans l'eau comprend le solvant et le produit en solution dans le solvant. Elle peut aussi être forme d'une microémulsion (ME), limpide, dont la phase dispersée dans l'eau comprend le solvant et le produit en solution dans le solvant.

Certains actifs phytosanitaires solides sont souvent difficiles à formuler. Par exemple le tebuconazole est un fongicide particulièrement efficace, et d'utilisation répandue, pour la culture du soja notamment. Pour certains actifs phytosanitaires, il est difficile de réaliser des formulations concentrées, faciles à diluer pour l'exploitant agricole, stables, et sans inconvénients (avérés ou perçus) substantiels en matière de sécurité, de toxicité et/ou d'eco-toxicité. Pour certains actifs, il est difficile de formuler à des concentrations relativement élevées, avec une stabilité suffisante. En particulier il est nécessaire d'éviter l'apparition de cristaux en particulier à basse température et/ou lors de la dilution et/ou lors du stockage à température élevée de la composition diluée. Les cristaux peuvent avoir des effets négatifs, notamment boucher les filtres des dispositifs utiliser pour répandre la composition diluée, boucher les dispositifs de pulvérisation, diminuer l'activité globale de la formulation, créer des problèmes inutiles de filières de déchets pour éliminer les cristaux, et/ou provoquer une mauvaise répartition du produit actif sur le champ agricole.

Les formulations comprenant le solvant présentent notamment:
- une solubilisation de quantités importantes d'actifs,
- une absence de cristallisation, même des conditions exigeantes,
- une bonne activité biologique pouvant être due à une bonne solvatation, et/ou
- un profile de sécurité, toxicologie et/ou eco-toxicologie perçu comme favorable.

La formulation phytosanitaire peut en outre être une formulation phytosanitaire concentrée comprenant:
a) un produit phytosanitaire actif,
b) le solvant
c) éventuellement au moins agent émulsifiant, de préférence un tensioactif, et
d) éventuellement de l'eau.

### Produit phytosanitaire actif a)

Des produits phytosanitaires actifs, notamment des produits non solubles dans l'eau et solides sont connus de l'homme du métier. Le produit phytosanitaire actif peut notamment être un herbicide, un insecticide, un acaricide, un fongicide, ou un agent d'élimination des rongeurs ("rodenticide" en anglais) par exemple un raticide.

A titre d'exemples non limitatifs de matières actives convenables, on peut citer entre autres l'Amétryne, le Diuron, le Linuron, le Chlortoluron, l'Isoproturon, le Nicosulfuron, le Metamitron, le Diazinon, l'Aclonifen, l'Atrazine, le Chlorothalonil, le Bromoxynil, le Bromoxynil heptanoate, le Bromoxynil octanoate, le Mancozeb, la Manèbe, le Zineb, la Phenmédipham, le Propanyl, la série des phénoxyphénoxy, la série des hétéroaryloxyphénoxy, le CMPP, le MCPA, le 2,4-D, la Simazine, les produits actifs de la série des imidazolinones, la famille des organophosphorés, avec notamment l'Azinphos-éthyl, l'Azinphos-méthyl, l'Alachlore, le Chlorpyriphos, le Diclofop-méthyl, le Fénoxapropp-éthyl, le Méthoxychlore, la Cyperméthrine, le Fenoxycarbe, le cymoxanil, le chlorothalonyl, lkes insecticides neonicotinoides, la famille des fongicide triazoles tels que l'azaconazole, bromuconazole, cyproconazole, difenoconazole, diniconazole, epoxyconazole, fenbuconazole, flusilazole, myclobutanyl, tebuconazole, triadimefon, triadimenol, des strobilurines telles que la pyraclostrobine, la picoxystrobine, l'azoxystrobine, la famoxadone, le kresoxym-methyl et la trifloxystrobine, les solfonylurées telles que le bensulfuron-methyl, le chlorimuron-ethyl, le chlorsulfuron, le metsulfuron-methyl, le nicosulfuron, le sulfomethuron-methyl, le triasulfuron, le tribenuron-methyl.

On choisi parmi cette liste les produits non-hydrosolubles.

On peut notamment mettre en oeuvre les produits phytosanitaires actifs suivants:

| | |
|---|---|
| Alachlor | 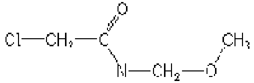 |
| | 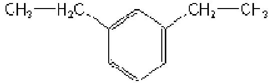 |
| Chlorpyrifos | 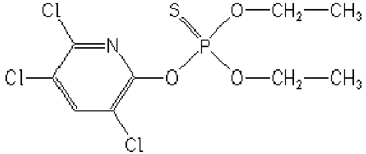 |
| alpha-cyperméthrine | 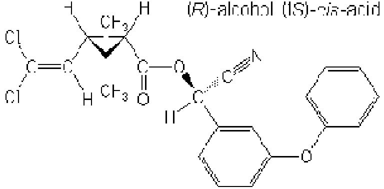 |
| | 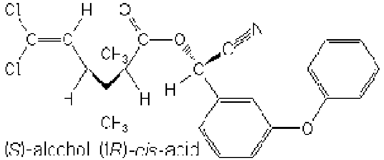 |
| | En mélange racémique et/ou en stéréoisomères isolés. |
| Phenmedipham | 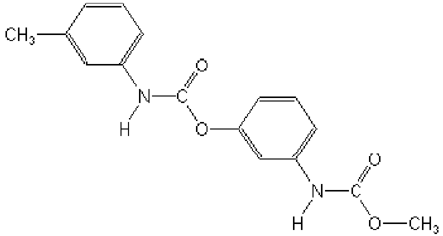 |
| Propanil | 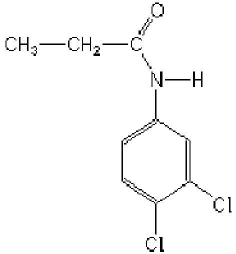 |
| Pendimethalin | 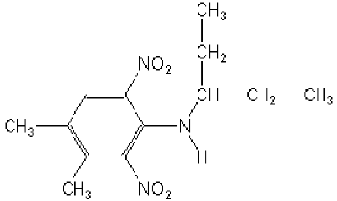 |
| triadimenol | 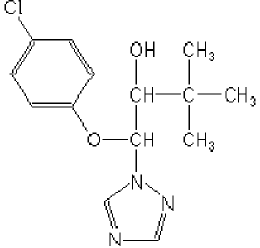 |
| Trifluralin | 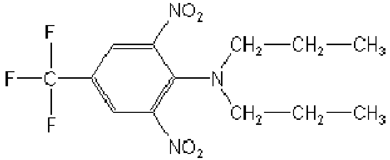 |
| Oxyfluorfen | 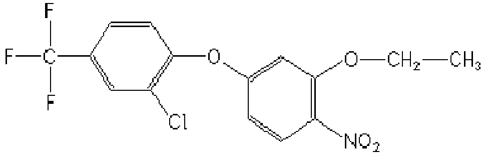 |
| Dimethoate | 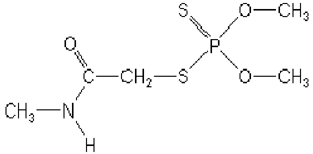 |
| Imidacloprid | 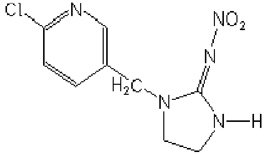 |
| Proxopur | 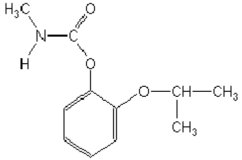 |
| Benomyl | 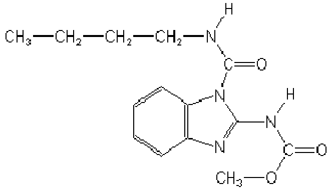 |
| Deltamethrine | 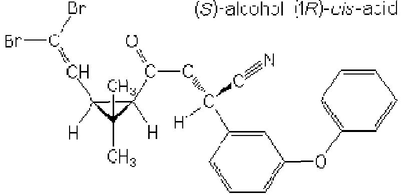 |
| Fenvalerate | 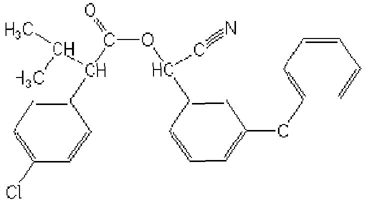 |
| Abamectin | 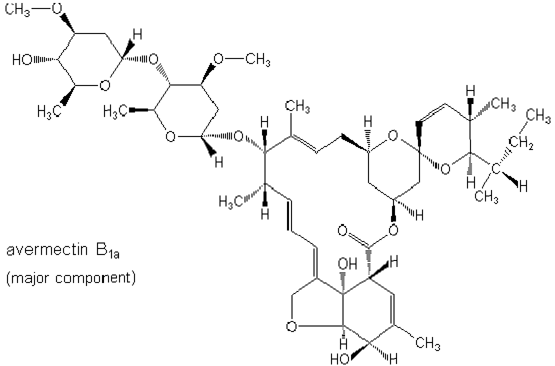 |
| | 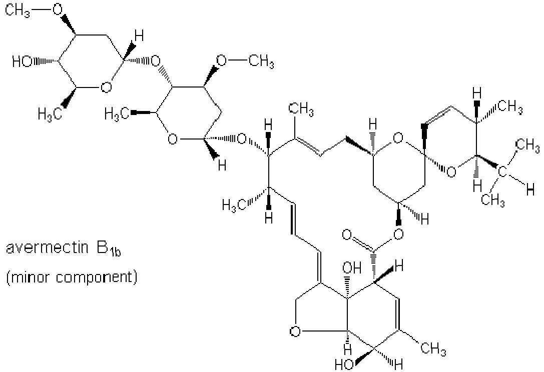 |
| Amicarbazone | 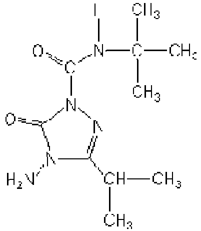 |
| Bifenthrin | 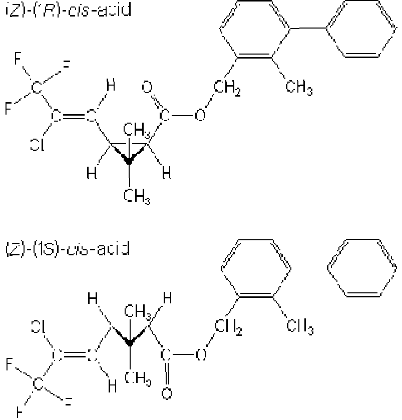 |
| Carbosulfan | 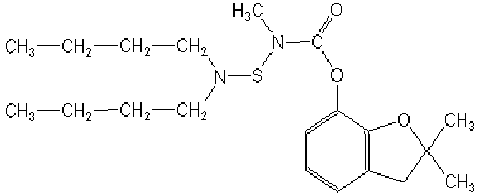 |
| Cyfluthrin | 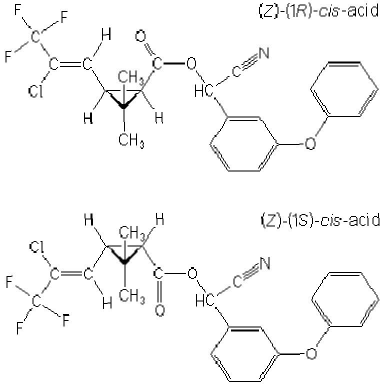 |
| Difenconazole | 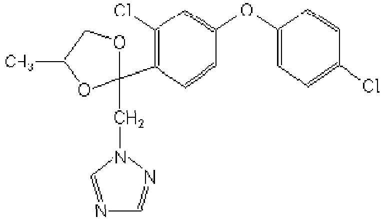 |
| Ethofenprox | 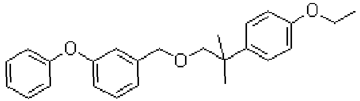 |
| Fenoxaprop-ethyl | 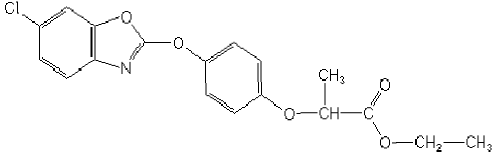 |
| Fipronil | 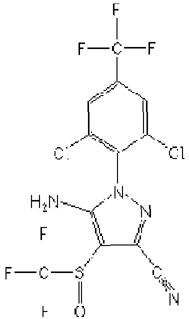 |
| Fenvalerate | 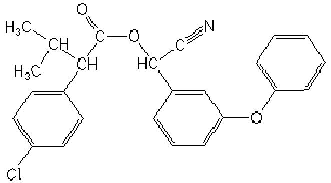 |
| Fluazifop-p-butyl | 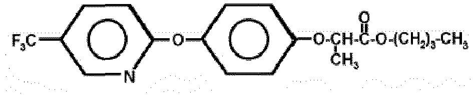 |
| Flufenouron | 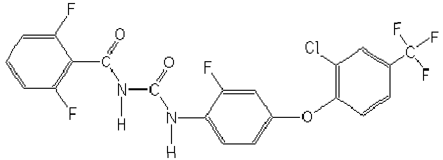 |
| Hexazinone | 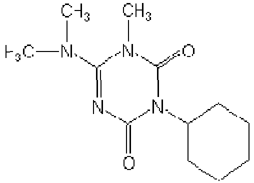 |
| Lambda-cyalothrin | 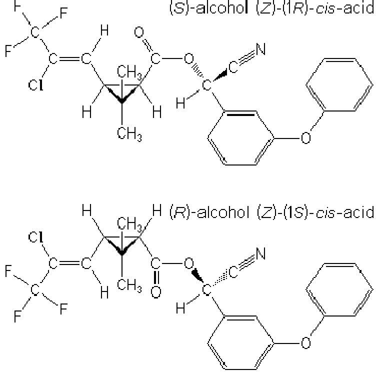 |
| Methomyl | 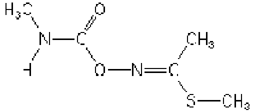 |
| Permethrin | 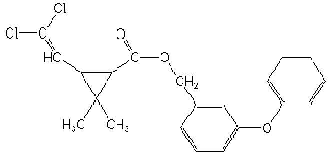 |
| Prochloraz | 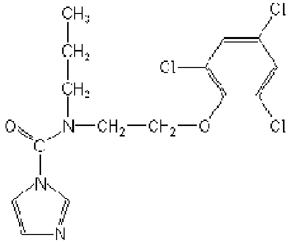 |
| Propiconazole | 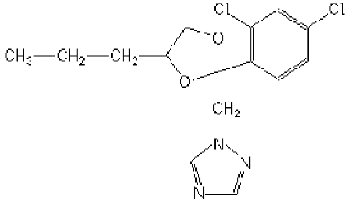 |
| Tebuconazole | 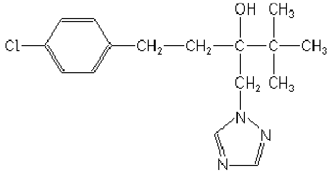 |

Ces produits et dénominations sont connus de l'homme du métier. On peut associer plusieurs produits phytosanitaires actifs.

### Agent émulsifiant c)

La formulation phytosanitaire peut comprendre un agent émulsifiant, typiquement et de préférence un tensioactif. Les agents émulsifiants sont des agents destinés à faciliter la mise en émulsion ou la dispersion après mise en présence de la formulation avec de l'eau, et/ou à stabiliser (dans le temps et/ou en température) l'émulsion ou la dispersion, par exemple en évitant une sédimentation.
Les tensioactifs sont des composés connus, qui présentent une masse molaire généralement relativement faible, par exemple inférieure à 1000 g/mol. Le tensioactif peut être un tensioactif anionique sous forme salifiée ou acide, non ionique de préférence polyalcoxylé, cationique, amphotère (terme incluant aussi les tensioactifs zwitterioniques). Il peut s'agir d'un mélange ou d'une association de ces tensioactifs.

A titre d'exemples de tensioactifs anioniques, on peut citer, sans intention de s'y limiter:
- les acides alkylsulfoniques, les acides arylsulfoniques, éventuellement substitués par un ou plusieurs groupements hydrocarbonés, et dont la fonction acide est partiellement ou totalement salifiée, comme les acides alkylsulfoniques en C₈-C₅₀, plus particulièrement en C₈-C₃₀, de préférence en C₁₀-C₂₂, les acides benzènesulfoniques, les acides naphtalènesulfoniques, substitués par un à trois groupements alkyles en C₁-C₃₀, de préférence en C₄-C₁₆, et/ou alcényles en C₂-C₃₀, de préférence en C₄-C₁₆.
- les mono- ou diesters d'acides alkylsulfosucciniques, dont la partie alkyle, linéaire ou ramifiée, éventuellement substituée par un ou plusieurs groupements hydroxylés et/ou alcoxylés, linéaires ou ramifiés en C₂-C₄ (de préférence éthoxylés, propoxylés, éthopropoxylés).
- les esters phosphates choisis plus particulièrement parmi ceux comprenant au moins un groupement hydrocarboné saturé, insaturé ou aromatique, linéaire ou ramifié, comprenant 8 à 40 atomes de carbone, de préférence 10 à 30, éventuellement substitués par au moins un groupement alcoxylé (éthoxylé, propoxylé, éthopropoxylé). En outre, ils comprennent au moins un groupe ester phosphate, mono- ou diestérifié de telle sorte que l'on puisse avoir un ou deux groupes acides libres ou partiellement ou totalement salifiés. Les esters phosphates préférés sont du type des mono- et diesters de l'acide phosphorique et de mono-, di- ou tristyrylphénol alcoxylé (éthoxylé et/ou propoxylé), ou de mono-, di- ou trialkylphénol alcoxylé (éthoxylé et/ou propoxylé), éventuellement substitué par un à quatre groupements alkyles ; de l'acide phosphorique et d'un alcool en C₈-C₃₀, de préférence en C₁₀-C₂₂ alcoxylé (éthoxylé ou éthopropoxylé); de l'acide phosphorique et d'un alcool en C₈-C₂₂, de préférence en C₁₀-C₂₂, non alcoxylé.
- les esters sulfates obtenus à partir d'alcools saturés, ou aromatiques, éventuellement substitués par un ou plusieurs groupements alcoxylés (éthoxylés, propoxylés, éthopropoxylés), et pour lesquels les fonctions sulfates se présentent sous la forme acide libre, ou partiellement ou totalement neutralisées. A titre d'exemple, on peut citer les esters sulfates obtenus plus particulièrement à partir d'alcools en C₈-C₂₀, saturés ou insaturés, pouvant comprendre 1 à 8 motifs alcoxylés (éthoxylés, propoxylés, éthopropoxylés); les esters sulfates obtenus à partir de phénol polyalcoxylé, substitués par 1 à 3 groupements hydroxycarbonés en C₂-C₃₀, saturés ou insaturés, et dans lesquels le nombre de motifs alcoxylés est compris entre 2 et 40 ; les esters sulfates obtenus à partir de mono-, di- ou tristyrylphénol polyalcoxylés dans lesquels le nombre de motifs alcoxylés varie de 2 à 40.

Les tensioactifs anioniques peuvent être sous forme acide (il sont potentiellement anioniques), ou sous une forme partiellement ou totalement salifiée, avec un contre-ion. Le contre-ion peut être un métal alcalin, tel que le sodium ou le potassium, un alcalino-terreux, tel que le calcium, ou encore un ion ammonium de formule N(R)₄⁺ dans laquelle R, identiques ou différents, représentent un atome d'hydrogène ou un radical alkyle en C₁-C₄ éventuellement substitué par un atome d'oxygène.

A titres d'exemples de tensioactifs non ioniques, on peut citer, sans intention de s'y limiter:
- les phénols polyalcoxylés (éthoxylés, propoxylés, éthopropoxylés)substitués par au moins un radical alkyle en C₄-C₂₀, de préférence en C₄-C₁₂, ou substitués par au moins un radical alkylaryle dont la partie alkyle est en C₁-C₆. Plus particulièrement, le nombre total de motifs alcoxylés est compris entre 2 et 100. A titre d'exemple, on peut citer les mono-, di- ou tri (phényléthyl) phénols polyalcoxylés, ou les nonylphénols polyalcoxylés. Parmi les di- ou tristyrylphenols éthoxylés et/ou propoxylés, sulfatés et/ou phosphatés, on peut citer, le di-(phényl-1 éthyl)phénol éthoxylé, contenant 10 motifs oxyéthylénés, le di-(phényl-1 éthyl)phénol éthoxylé, contenant 7 motifs oxyéthylénés, le di-(phényl-1 éthyl)phénol éthoxylé sulfaté, contenant 7 motifs oxyéthylénés, le tri-(phényl-1 éthyl)phénol éthoxylé, contenant 8 motifs oxyéthylénés, le tri-(phényl-1 éthyl)phénol éthoxylé, contenant 16 motifs oxyéthylénés, le tri-(phényl-1 éthyl)phénol éthoxylé sulfaté, contenant 16 motifs oxyéthylénés, le tri-(phényl-1 éthyl)phénol éthoxylé, contenant 20 motifs oxyéthylénés, le tri-(phényl-1 éthyl)phénol éthoxylé phosphaté, contenant 16 motifs oxyéthylénés.
- les alcools ou les acides gras en C₆-C₂₂, polyalcoxylés (éthoxylés, propoxylés, éthopropoxylés). Le nombre des motifs alcoxylés est compris entre 1 et 60. Le terme acide gras éthoxylé inclut aussi bien les produits obtenus par éthoxylation d'un acide gras par l'oxyde d'éthylène que ceux obtenus par estérification d'un acide gras par un polyéthylèneglycol.

- les triglycérides polyalcoxylés (éthoxylés, propoxylés, éthopropoxylés)d'origine végétale ou animale. Ainsi conviennent les triglycérides issus du saindoux, du suif, de l'huile d'arachide, de l'huile de beurre, de l'huile de graine de coton, de l'huile de lin, de l'huile d'olive, de l'huile de palme, de l'huile de pépins de raisin, de l'huile de poisson, de l'huile de soja, de l'huile de ricin, de l'huile de colza, de l'huile de coprah, de l'huile de noix de coco, et comprenant un nombre total de motifs alcoxylés compris entre 1 et 60. Le terme triglycéride éthoxylé vise aussi bien les produits obtenus par éthoxylation d'un triglycéride par l'oxyde d'éthylène que ceux obtenus par trans-estérification d'un triglycéride par un polyéthylèneglycol.
- les esters de sorbitan éventuellement polyalcoxylés (éthoxylés, propoxylés, éthopropoxylés), plus particulièrement les esters de sorbitol cyclisé d'acides gras de C₁₀ à C₂₀ comme l'acide laurique, l'acide stéarique ou l'acide oléique, et comprenant un nombre total de motifs alcoxylés compris entre 2 et 50.
Des émulsifiant utiles sont notamment les produits suivants, tous commercialisés par Rhodia:
- Soprophor® TSP/724: tensioactif à base de tristyrylphonol éthopropoxylé
- Soprophor® 796/O: tensioactif à base de tristyrylphonol éthopropoxylé
- Soprophor® CY 8: tensioactif à base de tristyrylphonol éthoxylé
- Soprophor® BSU: tensioactif à base de tristyrylphonol éthoxylé
- Alkamuls® RC: tensioactif à base d'huile de ricin éthoxylée
- Alkamuls® OR/36: tensioactif à base d'huile de ricin éthoxylée
- Alkamuls® T/20: tensioactif à base d'un ester de sorbitan

La formulation comprend avantageusement au moins 4%, de préférence au moins 5%, de préférence au moins 8%, en poids de matière sèche, d'au moins un tensioactif c).

On mentionne que le solvant peut être associé à un tensioactif aromatique et/ou non aromatique.

### Autres détails quant à la formulation phytosanitaire

La formulation phytosanitaire, concentrée, ne comprend de préférence pas des quantités importantes d'eau. Typiquement la teneur en eau est inférieure à 50% en poids, avantageusement inférieure à 25% en poids. Elle sera généralement inférieure à 10% en poids.

La formulation est de préférence un formulation liquide, par exemple sous forme d'un concentré emulsifiable (EC), d'une émulsion concentrée (EW) ou d'une micorémulsion (ME). Dans ce cas elle comprend de préférence moins de 500 g/L d'eau, plus préférablement moins de 250 g/L. Elle sera généralement inférieure à 100 g/L.

Les formulations peuvent avantageusement comprendre:
a) de 4 à 60%, de préférence de 10 à 50%, du produit phytosanitaire, en poids de matière active,
b) de 10 à 92%, de préférence de 20 à 80%, du solvant, en poids,
c) de 4 à 60%, de préférence de 5 à 50%, de préférence de 8 à 25%, en poids de matière sèche, d'un émulsifiant, de préférence d'un tensioactif,
d) de 0 à 10% en poids d'eau.

Il n'est pas exclu de réaliser des formulations solides, par exemple des formulations dans lesquelles un liquide comprenant le produit phytosanitaire solubilisé dans le solvant, est supporté par un minéral et/ou dispersé dans une matrice solide.

La formulation peut bien entendu comprendre d'autres ingrédients (ou "autres additifs") que le produit phytosanitaire actif, le(s) solvant(s), le(s) agent(s) émulsifiant(s) optionnel(s) et l'eau optionnelle. Elle peut notamment comprendre des agents de modification de la viscosité, des agents antimousse, notamment des antimousse siliconnés, des agents anti-rebond, des agents anti-lessivage, des charges inertes, notamment des charges minérales, des agents anti-gel...

Notamment les formulations peuvent comprendre des additifs, dits autres additifs, ne rentrant pas dans la définition des produits a), b), ou c), comme:
- d'autres solvants, généralement en faible quantité, c'est-à-dire en quantité inférieure au solvant du système solvant étant présent dans la plus faible quantité. Un solvant autre n'est pas entendu comme faisant partie du système solvant. A titre d'autres solvants on cite notamment les solvants de la famille des phosphates, phosphonates ou des oxydes de phosphines comme le TEBP, le TBP, le TEPO, le DBBP. On cite également les alkyldiméthyleamides où l'alkyl est en C₆-C₁₈, notamment ceux commercialisés sous la marque Genagen. On cite également les lactates d'esters, notamment ceux commercialisés sous la marque Purasolv. On cite également les esters méthyliques d'acides gras, notamment ceux commercialisés sous la marque Phytorobe. On cite également les diesters de diacides ("DiBasic Esters" en anglais), notamment ceux commercialisé par Rhodia sous les marques Rhodiasolv RPDE, et Rhodiasolv DIB. On cite également les coupes d'hydrocarbures, les amides cycliques comme la NMP, les lactones. On cite également les bis(dialkylamides) décrites dans le document WO 2008/074837.
- des inhibiteurs de cristallisation. Il peut s'agir des solvants mentionnés ci-dessus. Il peut également s'agir d'acides gras ou d'alcools gras non polyalkoxylés. On cite par exemple le produit Alkamuls® OL700 commercialisé par Rhodia.

Des procédés classiques de préparation de formulations phytosanitaires ou de mélanges de solvants peuvent être mis en oeuvre. On peut opérer par simple mélange des constituants.

La formulation phytosanitaire concentrée est destinée à être répandue sur un champ cultivé ou à cultiver, par exemple un champ de soja, le plus souvent après dilution dans de l'eau, pour obtenir une composition diluée. La dilution est généralement opérée par l'exploitant agricole, directement dans un réservoir ("tank-mix"), par exemple dans le réservoir d'un dispositif destiné à répandre la composition. Il n'est pas exclu que l'exploitant ajoute d'autres produits phytosanitaires, par exemple des fongicides, herbicides, pesticides, insecticides, des fertilisants. Ainsi, la formulation peut être utilisée pour préparer une composition diluée dans l'eau du produit phytosanitaire actif, par mélange d'au moins une part en poids de formulation concentrée avec au moins 10 parts d'eau, de préférence moins de 1000 parts. Les taux de dilution et les quantités à appliquer sur le champ dépendent généralement du produit phytosanitaire et de la dose souhaitable pour traiter le champ; cela peut être déterminé par l'exploitant agricole.
D'autres détails ou avantages pourront apparaître au vu des exemples qui suivent, sans caractère limitatif.

### EXEMPLES

### Exemple 1 - Préparation de CH₃-CH₂-CH(O-lsoAmHCH₂)-CONMe₂

La voie de synthèse est la suivante:

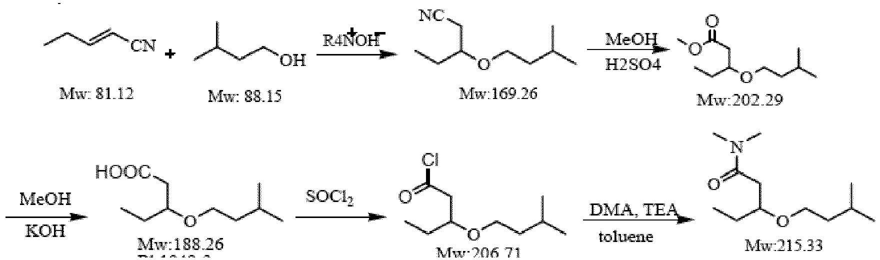

### Première étape :

450g (5,003 moles) d'alcool isoamylique, 10g (0,06 mole) de triton B (hydroxyde de benzyltriméthylammonium) et 414,1g (5,003 moles) de 2PN (2-pentène nitrile) sont chargés à température ambiante et maintenus agités à 35 +/- 4°C pendant 12 heures sous couverture d'azote.

609,7g de produit de stade 1 (3-isopentyloxy pentanenitrile) sont obtenus avec un rendement de 72% et une pureté de 98,8%.

### Deuxième étape :

1507 ml (37,44 moles) de méthanol, 350 ml (6,24 moles) d'acide sulfurique concentré de densité 1,84 et 543 g (3,12 moles) de produit de stade 1 sont mélangés et portés au reflux (80°C) sous agitation pendant 12 heures.

Lorsque la réaction est achevée, le milieu est versé sur 1500 ml d'eau glacée, puis extrait avec deux fois 300 ml d'acétate d'éthyle. Les phases organiques rassemblées sont lavées par une solution aqueuse d'hydrogénocarbonate de sodium jusqu'à pH 7-8.

Après évaporation de l'acétate d'éthyle, 515g de produit de stade 2 brut (3-isopentyloxy pentanoate de méthyle) sont obtenus, soit un rendement de 81,6%.

### Troisième étape :

623g (3,08 moles) de produite de stade 2 brut, 1750 ml (43,5 moles) de méthanol et 233g (3,39 moles) de potasse à 85% sont agités à 55°C pendant 12 heures.

Lorsque la réaction est achevée, le méthanol est évaporé et le milieu acidifié à pH 4-5 par une solution aqueuse diluée d'acide chlorhydrique.

Le milieu est extrait avec deux fois 500 ml d'acétate d'éthyle. Les phases organiques sont rassemblées et l'acétate d'éthyle est éliminé par évaporation, conduisant à 520g, soit 90% de rendement en produite de stade 3 (acide 3-isopentyloxy pentanoique) avec une pureté de 98%.

### Quatrième étape :

372 g (1,94 moles) de stade 3 brut et 465g (3,89 moles) de chlorure de thionyle sont agités à 55°C pendant 4 heures. Lorsque la réaction est achevée, le chlorure de thionyle est évaporé conduisant à 380g, soit un rendement de 95,7% en produit de stade 4 (chlorure de l'acide 3-isopentyloxy pentanoique), ayant une pureté de 95,5%.

### Cinquième étape :

2500 ml de toluène, 450 ml (3,23 moles) de triéthylamine, 280 ml (4,22 moles) de dimethylamine et 510g (2,36 moles) de produite de stade 4 brut sont mélangés à température ambiante pendant 15 heures.

Lorsque la réaction est achevée, le chlorure de triéthylammonium formé est éliminé par filtration et lavé avec deux fois 300 ml de toluène. Les phases toluèniques sont rassemblées et le toluène est éliminé par évaporation.

Le produit est ensuite lavé avec une solution aqueuse d'hydrogénocarbonate de sodium jusqu'à pH 7-8 et extrait à l'acétate d'éthyle.

L'acétate d'éthyle est éliminé par évaporation et le produit purifié par distillation fractionnée (température d'ébullition : 105°C/100 Pa).

On obtient 400g de produit de stade 5, 3-isopentyloxy N,N-diméthylpentanamide, soit un rendement de 79% avec une pureté de 98,3%

### Exemple 2 - Préparation de CH₃-CH₂-CH(OMe)-CH₂)-CONMe₂

On opère de manière similaire à l'exemple 1 en remplaçant l'alcool isoamylique par du méthanol.

### Exemple 3 - Préparation de CH₃-CH₂-CH(OCyclo)-(CH₂)-CONMe₂

La voie de synthèse est la suivante:

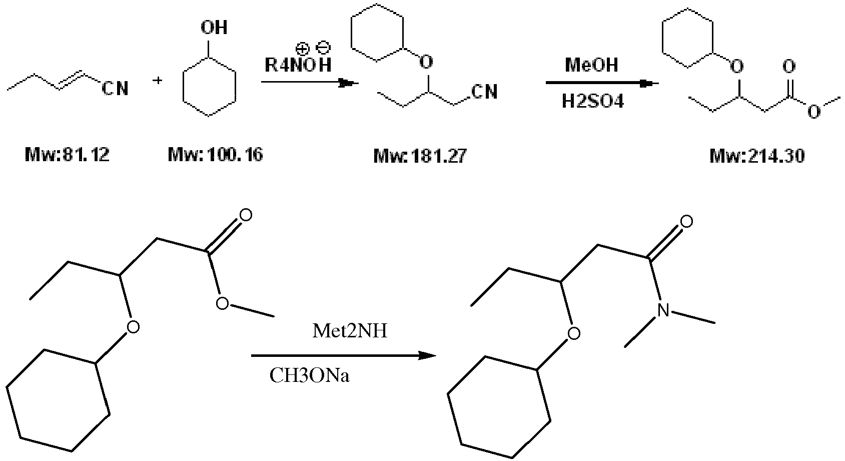

### Première étape :

1100mL (10,200 moles) de cyclohexanol, 28g (0,067 mole) de triton B (hydroxyde de benzyltriméthylammonium) sont chargés sous couverture d'azote. 800g (9,862 moles) de 2PN (2-pentène nitrile) sont chargés lentement en maintenant la température à 20 +/- 5°C puis le mélange est maintenu à température ambiante pendant 38 heures tout en suivant la réaction par chromatographie gaz. La conversion obtenue est environ de 60% pour le composé principal. Le catalyseur est neutralisé par l'ajout d'acide acétique puis l'intermédiaire est isolé par distillation sous pression réduite.

560g de produit de stade 1 (3-cyclohexanoxy pentanenitrile) sont obtenus avec une pureté supérieure à 90%.

### Deuxième étape :

1325 ml (33,10 moles) de méthanol, 358 ml (6,62 moles) d'acide sulfurique concentré de densité 1,84 et 600 g (3,31 moles) de produit de stade 1 sont mélangés et portés au reflux (80°C) sous agitation pendant 12 heures.

Lorsque la réaction est achevée, le milieu est versé sur 1500 ml d'eau glacée, puis extrait avec deux fois 300 ml d'acétate d'éthyle. Les phases organiques rassemblées sont lavées par une solution aqueuse d'hydrogénocarbonate de sodium jusqu'à pH 7-8.

Après évaporation de l'acétate d'éthyle, 534g de produit de stade 2 brut (3- cyclohexyloxy pentanoate de méthyle) sont obtenus. Cet intermédiaire est alors distillé sous pression réduite pour obtenir 497g de produit ayant une pureté supérieure à 90% soit un rendement de 70,2%.

### Troisième étape

Dans un réacteur de 1L sont chargés 284,5 g d'une solution de NN-diméthyl amine à 46 % (2,93 moles de DMA) dans le méthanol. Cette solution est refroidie à 0°C +/- 2°C.

Le condenseur du réacteur est alimenté par de l'eau glycolée à 2°C.

Sur la solution de NN-diméthyl amine dans le méthanol sont introduits
- 573 g de 3- cyclohexyloxy pentanoate (2,68 moles) de méthyle obtenus à l'issu de la deuxième étape
- 22,9 g de solution CH3ONa dans le méthanol (titre de la solution 25 % massique).

L'introduction du CH3ONa est réalisée en 5 minutes sous forte agitation.

La température de la masse réactionnelle est portée en 2 heures à la température de 25°C.

L'avancement de la réaction est suivi par analyse GC.

**Taux de transformation %**

| | |
|---|---|
| Maintien 3H à 25°C | 31,67 |
| Maintien 27H à 25°C | 68,33 |
| Maintien 46H à 25°C | 72,75 |
| Maintien 118H à 25°C | 93,78 |

Des ajouts de CH3ONa et de NN-diméthyl amine sont réalisés aux temps suivants :

| Temps (h) | Ajout |
|---|---|
| 29 | 32,7 g de DMA dans le méthanol (titre 37,5 %) |
| 46 | 79,7 g de DMA (introduction à 0°C) |
| 52 | 5 g de CH3ONa dans le méthanol (titre de la solution 25 % massique). |

### Isolement du produit

On opère les traitements détaillés ci-dessous:
Elimination de la DMA:
   L'excès de DMA et une partie du méthanol sont distillés sous pression réduite (P < 30 mbar) à une température inférieure à 25°C.
   - masse distillée 293 g.
Neutralisation du catalyseur:
   - ajout de 50 g d'eau puis de 356 g de solution aqueuse d'acide chlorhydrique (3,4 %) . Le pH final de la phase aqueuse est de 7,01.

La phase aqueuse est décantée, la phase organique est lavée par 300 g d'eau.

### Séchage de la phase organique

La phase organique est additionnée de 120 g de cyclohexane et concentrée sous vide partiel (pression < 21 mbar / température de masse < 50°C).

Masse réactionnelle obtenue : 533,8 g d'une solution jaune pale. Sa composition est la suivante:
- CH₃-CH₂-CH(OCyclo)-(CH₂)-CONMe₂ : 87,69%
- 3-cyclohexyloxy pentanoate de méthyle: 7,43%
- Impuretés 4,88%
Le rendement molaire est de 76 %.

### Purification

987 g de masse réactionnelle obtenue selon le protocole ci-dessus sont purifiés par étêtage.

Conditions d'étêtage :
- pression < 2 mbar
- température de masse de 25 à 90°C
- température des distillats 25 à 76°C
- masse distillée 240 g

Masse réactionnelle obtenue : 729 g d'une solution jaune pale. Elle est composée à 93% de CH₃-CH₂-CH(OCyclo)-(CH₂)-CONMe₂

### Exemples 4 à 7 - Utilisations comme solvants - Formulations phytosanitaires

Par mélange des ingrédients, on prépare des formulations de divers actifs phytosanitaires, de type concentré émulsionnable (EC).

Les formulations comprennent:
- l'actif, en quantité en poids (de matière active) indiquée dans le tableau ci-dessous,
- 10% en poids de tensioactif Alkamuls® RC, commercialisé par Rhodia
- et, comme solvant, le reste de composé des exemples.

Les exemples 4 sont des exemples comparatifs où est utilisé comme solvant le produit Rhodiasolv® ADMA10, Rhodia (zone Asie Pacifique): Solvant alkyldiméthylamide.

On effectue les tests suivants:
- Observation visuelle à 25°C - On note l'aspect de la formulation et on repère éventuellement la présence de cristaux
- Observation visuelle à 0°C - On place la formulation pendant 7 jours à 0°C et on note l'aspect de la formulation et on repère éventuellement la présence de cristaux (test CIPAC MT39)
- Observation visuelle à 0°C avec nucléation: On introduit un cristal de la matière active dans la formulation ayant passé 7 jours à 0°C pour nucléation, et on place à nouveau la formulation pendant 7 jours à 0°C. On note l'aspect de la formulation et on repère éventuellement la présence de cristaux.

| *Exemple* | *Solvant* | *Actif* | *Apparence à 25°C* | *Apparence à 0°C* | *Apparence à 0°C avec nucléation* |
|---|---|---|---|---|---|
| 4.1C | Rhodiasolv® ADMA 10 | Alachlor - 48% | Limpide | Cristaux | Cristaux |
| 4.5C | Rhodiasolv® ADMA 10 | Triadimenol 23% | Limpide | Limpide | Cristaux |
| 4.6C | Rhodiasolv® ADMA 10 | Trifluralin 40% | Limpide | Limpide | Cristaux |
| 4.7C | Rhodiasolv® ADMA 10 | Oxyfluorfen 22% | Limpide | Limpide | Cristaux |
| 4.11 C | Rhodiasolv® ADMA10 | Propoxur 20% | Limpide | Limpide | Cristaux |
| 5.1 | Exemple 1 | Alachlor - 48% | Limpide | Limpide | Cristaux |
| 5.2 | Exemple 1 | Phenmedipham - 16% | Limpide | Limpide | Limpide |
| 5.3 | Exemple 1 | Propanil - 36% | Limpide | Limpide | Limpide |
| 5.4 | Exemple 1 | Tebuconazole - 25% | Limpide | Limpide | Limpide |
| 5.9 | Exemple 1 | Alpha-cypermethrine 10% | Limpide | Limpide | Limpide |
| 6.1 | Exemple 2 | Alachlor - 48% | Limpide | Limpide | Cristaux |
| 6.3 | Exemple 2 | Propanil - 36% | Limpide | Limpide | Limpide |
| 6.4 | Exemple 2 | Tebuconazole - 25% | Limpide | Limpide | Limpide |
| 6.5 | Exemple 2 | Triadimenol 23% | Limpide | Limpide | Limpide |
| 6.6 | Exemple 2 | Trifluralin 40% | Limpide | Limpide | Limpide |
| 6.7 | Exemple 2 | Oxyfluorfen 22% | Limpide | Limpide | Limpide |
| 6.8 | Exemple 2 | Chlorpyrifos 40% | Limpide | Limpide | Limpide |
| 6.9 | Exemple 2 | Alpha-cypermethrine 10% | Limpide | Limpide | Limpide |
| 6.11 | Exemple 2 | Propoxur 20% | Limpide | Limpide | Limpide |
| 7.1 | Exemple 3 | Alachlor - 48% | Limpide | Limpide | Limpide |
| 7.2 | Exemple 3 | Phenmedipham 16% | Limpide | Limpide | Limpide |
| 7.3 | Exemple 3 | Propanil - 36% | Limpide | Limpide | Limpide |
| 7.4 | Exemple 3 | Tebuconazole 25% | Limpide | Limpide | Limpide |
| 7.5 | Exemple 3 | Triadimenol 23% | Limpide | Limpide | Limpide |
| 7.6 | Exemple 3 | Trifluralin 40% | Limpide | Limpide | Limpide |
| 7.7 | Exemple 3 | Oxyfluorfen 22% | Limpide | Limpide | Limpide |
| 7.8 | Exemple 3 | Chlorpyrifos 40% | Limpide | Limpide | Limpide |
| 7.9 | Exemple 3 | Alpha-cypermethrine 10% | Limpide | Limpide | Limpide |
| 7.10 | Exemple 3 | Difenconazole 25% | Limpide | Limpide | Limpide |
| 7.11 | Exemple 3 | Propoxur 20% | Limpide | Limpide | Limpide |

### Exemple 8 - Formulation de Tebuconazole

On prépare la formulation EC suivante:

| | |
|---|---|
| Tebuconazole tech.97% | 258 g/L |
| Solvant de l'exemple 3 | 608 g/L |
| Geronol® TBE-724 (tensioacitf, Rhodia) | 150 g/L |

On évalue les propriétés de la formulation après la préparation:
- Densité à 20°C: 1,016
- pH (solution à 5%): 6,3
- Emulsification (test Cipac, à 1% de concentration à 30°C, après 24 heures

| | | |
|---|---|---|
| A | D | C |
| 0t | 0 | 0 |

- Emulsification (test Cipac, à 5% de concentration à 30°C, après 2 heures

| | | |
|---|---|---|
| A | D | C |
| 0t | 0 | 0 |

- Apparence à 0°C: Solution voilée, devenant limpide à la température ambiante
- Apparence à 54°C: Solution limpide

On évalue les propriétés de la formulation après 14 jours à 54°C:
- pH (solution à 5%): 5,1
- Emulsification (test Cipac, à 1% de concentration à 30°C, après 4 heures

| | | |
|---|---|---|
| A | D | C |
| 0t | 0 | 0 |

- Emulsification (test Cipac, à 5% de concentration à 30°C, après 4 heures

| | | |
|---|---|---|
| A | D | C |
| 0t | 0 | 0 |

- Apparence à 0°C: Solution voilée, devenant limpide à la température ambiante
- Apparence à 54°C: Solution limpide

## Revendications

1. Composé de formule (I) suivante:
R^{a}R^{b}C(OR¹)-CHR^{c}-CONR²R³ (I)
où
- R^{a}, R^{b} et R^{c}, identiques ou différents, sont des groupes choisis parmi l'atome d'hydrogène et les groupes alkyles linéaires ou ramifiés, au moins un des groupes choisis parmi R^{a}, R^{b} et R^{c} étant différent de l'atome d'hydrogène,
- R¹ est choisi parmi les groupes méthyle, éthyle, propyle, isopropyle, n-butyle, isobutyle, n-pentyle, isopentyle, isoamyle, n-hexyle, cyclohexyle, n-octyle, isooctyle, 2-ethylhexyle ou tridécyle ; - R² et R³, identiques ou différents, sont des groupes choisis parmi les groupes méthyle, éthyle, n-propyle, isopropyle, n-butyle, isobutyle, n-pentyle, amyle, isoamyle, hexyle ou cyclohexyle.

2. Composé selon la revendication 1, **caractérisé en ce que** le nombre total d'atomes de carbone, hors groupes R¹, R² et R³ est de 4, 5, ou 6.

3. Composé selon l'une des revendications précédentes, **caractérisé en ce que**:
- R^{c}= H, et R^{b} = H, ou
- R^{c} = Me et R^{b} = H, ou
- R^{c} = H et R^{b} = Me.

4. Composé selon l'une des revendications précédentes, **caractérisé en ce que** R^{a} est un groupe méthyle ou éthyle.

5. Composé selon l'une des revendications précédentes, **caractérisé en ce que**
- R^{c}= H, et R^{b} = H, et R^{a} = Et, ou
- R^{c} = Me et R^{b} = H et R^{a} = Me, ou
- R^{c} = H et R^{b} = Me et R^{a} = Me.

6. Composé selon l'une des revendications précédentes, **caractérisé en ce que** R² et R³ sont des groupes méthyle.

7. Composé selon l'une des revendications précédentes, **caractérisé en ce qu'**il présente l'une des formules suivantes:

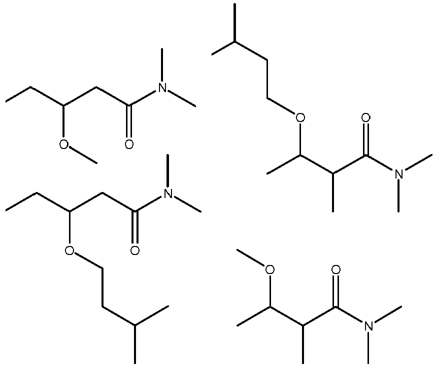

8. Procédé de préparation d'un composé selon l'une des revendications précédentes, par transformation à partir d'un alcène-nitrile de formule (I')
R^{a}R^{b}C=CR^{c}-CN (I').

9. Procédé selon la revendication 8, **caractérisé en ce que** la transformation comprend les étapes suivantes:
étape 1) on réagit l'alcène-nitrile de formule (I') avec un alcool de formule R¹-OH pour obtenir un éther-nitrile de formule (II')
R^{a}R^{b}C(OR¹)-CHR^{c}-CN (II')
étape 2) on transforme le groupe -CN de l'éther-nitrile en groupe amide de manière à obtenir le produit de formule (I).

10. Procédé selon la revendication 9, **caractérisé en ce que** l'étape 2) comprend les étapes suivantes :
étape 2a): on transforme le groupe -CN en groupe -COOH ou -COOR' où R' est un alkyle en C₁-C₄,
étape 2b): on transforme le groupe -COOH ou -COOR' en groupe -CONR²R³ à l'aide d'une amine de formule HNR²R³, de manière à obtenir le composé de formule (I).

11. Procédé selon la revendication 10, **caractérisé en ce que** l'étape 2) comprend les étapes suivantes:
2a') on transforme le groupe -CN en groupe -COOH directement par hydrolyse ou en formant un groupe -COOR' puis en hydrolysant,
2b') on transforme le groupe -COOH en groupe -CONR²R³ directement par réaction avec une amine de formule HNR²R³ ou en formant un groupe -COCl puis en réagissant avec une amine de formule HNR²R³, de manière à obtenir le composé de formule (I).

12. Procédé selon la revendication 10, **caractérisé en ce que** l'étape 2) comprend les étapes suivantes:
2a") on transforme le groupe -CN en groupe -COOR'
2b") on transforme le groupe -COOR' en groupe -CONR²R³ à l'aide d'une amine de formule HNR²R³, de manière à obtenir le composé de formule (I).

13. Utilisation du composé selon l'une des revendications 1 à 7, comme tensioactif, solvant, co-solvant, décapant, inhibiteur de cristallisation, agent nettoyant, agent de dégraissage, agent plastifiant ou agent de coalescence.

## Patentansprüche

1. Verbindung mit der folgenden Formel (I):
R^{a}R^{b}C(OR¹)-CHR^{c}-CONR²R³ (I),
wobei:
R^{a}, R^{b} und R^{c}, die gleich oder verschieden sind, Gruppen sind, ausgewählt aus einem Wasserstoffatom und linearen oder verzweigten Alkylgruppen, wobei mindestens eine der aus R^{a}, R^{b} und R^{c} ausgewählten Gruppen von einem Wasserstoffatom verschieden ist,
R¹ ausgewählt ist aus den Gruppen Methyl, Ethyl, Propyl, Isopropyl, n-Butyl, Isobutyl, n-Pentyl, Isopentyl, Isoamyl, n-Hexyl, Cyclohexyl, n-Octyl, Isooctyl, 2-Ethylhexyl oder Tridecyl;
R² und R³, die gleich oder verschieden sind, Gruppen sind, ausgewählt aus den Gruppen Methyl, Ethyl, n-Propyl, Isopropyl, n-Butyl, Isobutyl, n-Pentyl, Amyl, Isoamyl, Hexyl oder Cyclohexyl.

2. Verbindung nach Anspruch 1,
**dadurch gekennzeichnet, dass** die Gesamtanzahl von Kohlenstoffatomen, außerhalb der Gruppen R¹, R² und R³, 4, 5 oder 6 beträgt.

3. Verbindung nach einem der vorhergehenden Ansprüche,
**dadurch gekennzeichnet, dass**:
R^{c} = H, und R^{b} = H, oder
R^{c} = Me, und R^{b} = H, oder
R^{c} = H, und R^{b} = Me.

4. Verbindung nach einem der vorhergehenden Ansprüche,
**dadurch gekennzeichnet, dass** R^{a} eine Gruppe Methyl oder Ethyl ist.

5. Verbindung nach einem der vorhergehenden Ansprüche,
**dadurch gekennzeichnet, dass**:
R^{c} = H, und R^{b} = H, und R^{a} = Et, oder
R^{c} = Me, und R^{b} = H, und R^{a} = Me, oder
R^{c} = H, und R^{b} = Me, und R^{a} = Me.

6. Verbindung nach einem der vorhergehenden Ansprüche,
**dadurch gekennzeichnet, dass** R² und R³ Methylgruppen sind.

7. Verbindung nach einem der vorhergehenden Ansprüche,
**dadurch gekennzeichnet, dass** diese eine der folgenden Formeln aufweist:

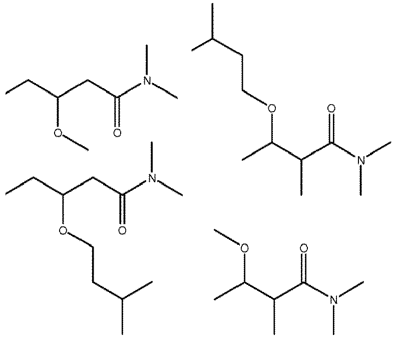

8. Verfahren zur Herstellung einer Verbindung nach einem der vorhergehenden Ansprüche, durch Überführen aus einem Alkennitril mit der Formel (I'):
R^{a}R^{b}C=CR^{c}-CN (I').

9. Verfahren nach Anspruch 8,
**dadurch gekennzeichnet, dass** das Überführen die folgenden Schritte umfasst:
Schritt 1) das Alkennitril mit der Formel (I') wird mit einem Alkohol mit der Formel R¹-OH umgesetzt, wobei ein Ethernitril mit der Formel (II') erhalten wird:
R^{a}R^{b}C(OR¹)-CHR^{c}-CN (II'),
Schritt 2) die Gruppe -CN des Ethernitrils wird in eine Amidgruppe übergeführt, wobei das Produkt mit der Formel (I) erhalten wird.

10. Verfahren nach Anspruch 9,
**dadurch gekennzeichnet, dass** der Schritt 2) die folgenden Schritte umfasst:
Schritt 2a): die Gruppe -CN wird in die Gruppe -COOH oder -COOR' übergeführt, wobei R' ein C₁-C₄-Alkyl ist,
Schritt 2b): die Gruppe -COOH oder -COOR' wird in die Gruppe - CONR²R³ mit Hilfe eines Amins mit der Formel HNR²R³ übergeführt, wobei die Verbindung mit der Formel (I) erhalten wird.

11. Verfahren nach Anspruch 10,
**dadurch gekennzeichnet, dass** der Schritt 2) die folgenden Schritte umfasst:
2a') die Gruppe -CN wird in die Gruppe -COOH direkt durch Hydrolyse oder durch Bildung einer Gruppe -COOR', dann Hydrolyse, übergeführt,
2b') die Gruppe -COOH wird in die Gruppe -CONR²R³ direkt durch Umsetzen mit einem Amin mit der Formel HNR²R³ oder durch Bildung einer Gruppe -COCl, dann Umsetzen mit einem Amin mit der Formel HNR²R³, übergeführt, wobei die Verbindung mit der Formel (I) erhalten wird.

12. Verfahren nach Anspruch 10,
**dadurch gekennzeichnet, dass** der Schritt 2) die folgenden Schritte umfasst:
2a") die Gruppe -CN wird in die Gruppe -COOR' übergeführt,
2b") die Gruppe -COOR' wird in die Gruppe -CONR²R³ mit Hilfe eines Amins mit der Formel HNR²R³ übergeführt, wobei die Verbindung mit der Formel (I) erhalten wird.

13. Verwendung der Verbindung nach einem der Ansprüche 1 bis 7, als Tensid, Lösungsmittel, Co-Lösungsmittel, Ätzmittel, Kristallisationsinhibitor, Reinigungsmittel, Entfettungsmittel, Weichmacher oder Koaleszenzmittel.

## Claims

1. Compound of following formula (I):
R^{a}R^{b}C(OR¹)-CHR^{c}-CONR²R³ (I)
where
- R^{a}, R^{b} and R^{c}, which are identical or different, are groups chosen from the hydrogen atom and linear or branched alkyl groups, at least one of the groups chosen from R^{a}, R^{b} and R^{c} being other than the hydrogen atom,
- R¹ is chosen from methyl, ethyl, propyl, isopropyl, n-butyl, isobutyl, n-pentyl, isopentyl, isoamyl, n-hexyl, cyclohexyl, n-octyl, isooctyl, 2-ethylhexyl or tridecyl groups;
- R² and R³, which are identical or different, are groups chosen from methyl, ethyl, n-propyl, isopropyl, n-butyl, isobutyl, n-pentyl, amyl, isoamyl, hexyl or cyclohexyl groups.

2. Compound according to Claim 1, **characterized in that** the total number of carbon atoms, excluding the R¹, R² and R³ groups, is 4, 5 or 6.

3. Compound according to either of the preceding claims, **characterized in that**:
- R^{c} = H and R^{b} = H, or
- R^{c} = Me and R^{b} = H, or
- R^{c} = H and R^{b} = Me.

4. Compound according to one of the preceding claims, **characterized in that** R^{a} is a methyl or ethyl group.

5. Compound according to one of the preceding claims, **characterized in that**:
- R^{c} = H and R^{b} = H and R^{a} = Et, or
- R^{c} = Me and R^{b} = H and R^{a} = Me, or
- R^{c} = H and R^{b} = Me and R^{a} = Me.

6. Compound according to one of the preceding claims, **characterized in that** R² and R³ are methyl groups.

7. Compound according to one of the preceding claims, **characterized in that** it exhibits one of the following formulae:

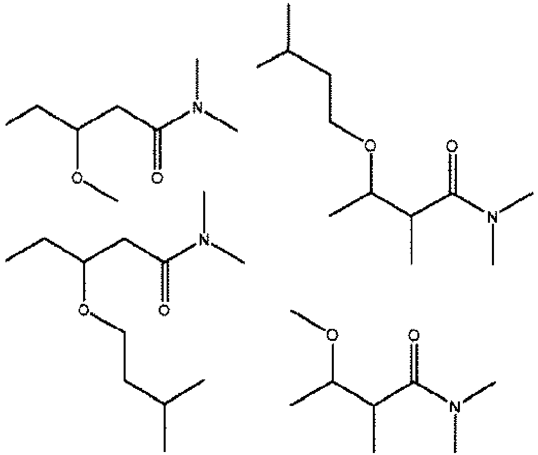

8. Process for the preparation of a compound according to one of the preceding claims, by conversion starting from an alkenenitrile of formula (I')
R^{a}R^{b}C=CR^{c}-CN (I').

9. Process according to Claim 8, **characterized in that** the conversion comprises the following stages:
stage 1) the alkenenitrile of formula (I') is reacted with an alcohol of formula R¹-OH in order to obtain an ether-nitrile of formula (II')
R^{a}R^{b}C(OR¹)-CHR^{c}-CN (II')
stage 2) the -CN group of the ether-nitrile is converted to the amide group, so as to obtain the product of formula (I).

10. Process according to Claim 9, **characterized in that** stage 2) comprises the following stages:
stage 2a): the -CN group is converted to the -COOH or -COOR' group, where R' is a C₁-C₄ alkyl,
stage 2b): the -COOH or -COOR' group is converted to the -CONR²R³ group using an amine of formula HNR²R³, so as to obtain the compound of formula (I).

11. Process according to Claim 10, **characterized in that** stage 2) comprises the following stages:
2a') the -CN group is converted to the -COOH group directly by hydrolysis or by forming a -COOR' group and by then hydrolyzing,
2b') the -COOH group is converted to the -CONR²R³ group directly by reaction with an amine of formula HNR²R³ or by forming a -COCl group and by then reacting with an amine of formula HNR²R³, so as to obtain the compound of formula (I).

12. Process according to Claim 10, **characterized in that** stage 2) comprises the following stages:
2a") the -CN group is converted to the -COOR' group,
2b") the -COOR' group is converted to the -CONR²R³ group using an amine of formula HNR²R³, so as to obtain the compound of formula (I).

13. Use of the compound according to one of Claims 1 to 7 as surfactant, solvent, cosolvent, stripping agent, crystallization inhibitor, cleaning agent, degreasing agent, plasticizing agent or coalescence agent.
